# EUROPEAN PATENT SPECIFICATION

(11) **EP 4 277 633 B1**
(45) Date of publication and mention of the grant of the patent: **11.11.2026**
(21) Application number: 22701191.3
(22) Date of filing: 13.01.2022
(51) Int. Cl.: A61K 31/665, A61K 31/00, A61P 17/02

(54) **CYCLIC OXAPHOSPHINANE COMPOUNDS AND ANALOGUES THEREOF FOR TREATING FIBROTIC DISEASES**
ZYKLISCHE OXAPHOSPHINAN-VERBINDUNGEN UND DEREN ANALOGA ZUR BEHANDLUNG VON FIBROTISCHEN ERKRANKUNGEN
COMPOSÉS D'OXAPHOSPHINANE CYCLIQUE ET ANALOGUES DE CEUX-CI POUR LE TRAITEMENT DES MALADIES FIBROTIQUES

(30) Priority: 13.01.2021 EP 21305031
(43) Date of publication of application: 22.11.2023
(73) Proprietor: Phost'in Therapeutics, 34090 Montpellier (FR); RD Nephrologie, 34000 Montpellier (FR)
(72) Inventor: CLARION, Ludovic, 34980 Combailleux (FR); CHORRO, Karine, 34830 Clapiers (FR); LOISEAU, Séverine, 34570 Vailhauques (FR); PAU, Bernard, 34000 Montpellier (FR); ARGILES, Angel, 34980 Saint Gely Du Fesc (FR); GAYRARD, Nathalie, 34090 Montpellier (FR)
(74) Representative: Cabinet Becker et Associés
(86) International application number: PCT/EP2022/050652
(87) International publication number: WO 2022/152795

(56) References cited:
- WO-A1-2009/004096
- WO-A1-2014/128429
- HANESSIAN S ET AL: "Synthesis of a phostone glycomimetic of the endothelin converting enzyme inhibitor phosphoramidon", BIORGANIC & MEDICINAL CHEMISTRY LETTERS, ELSEVIER, AMSTERDAM , NL, vol. 9, no. 16, 16 August 1999 (1999-08-16), pages 2441 - 2446, XP004174206, ISSN: 0960-894X, DOI: 10.1016/S0960-894X(99)00401-1

## Description

### TECHNICAL FIELD

The present invention relates to the use of heterocyclic phosphonic compounds or compositions comprising the same for treating fibrotic diseases.

### BACKGROUND

Under normal circumstances any organ or tissue of the organism reacts to injury by a reparative process aiming at restoration of the functional integrity of the injured tissue. The reactions involved in tissue repair are self-limited and stop when completing wound healing. Under pathologic circumstances, the reactions involved in tissue repair escape the regulatory mechanisms and evolve into an uncontrolled wound healing response, leading to fibrotic process. It results in protracted and progressive accumulation of an excess of fibrous material that alters and disorganizes the normal organ architecture and function. The deposition and accumulation of excess extracellular matrix (ECM) components such as collagen and fibronectin results in the hardening and scarring of tissues, cause an abnormal remodeling of the organ, and may ultimately lead to organ failure in fibrotic diseases.

Wounding in solid organs typically involves endothelial damage, platelet aggregation and activation eliciting an inflammatory response with infiltration of neutrophils, macrophages, eosinophils and lymphocytes to the wound site. Infiltrating inflammatory cells and affected epithelial cells secrete a variety of growth factors and cytokines that serve to further amplify the inflammatory response. Molecules such as TGF-β, PDGF and IL-13 activate macrophages and lead to the recruitment, proliferation and activation of fibroblasts at the wound site. Activated fibroblasts or myofibroblasts express smooth muscle actin and secrete collagen and other ECM components that stabilize the cellular substratum. This allows proliferation and migration of epithelial and endothelial cells over the temporary matrix to regenerate the damaged tissue. Once completed, the inflammatory process shuts down while fibroblasts undergo apoptosis leading to resolution of the wound response.

In fibrotic remodeling, persistent tissue insult or injury, or a dysregulation of the repair mechanisms, leads to an inappropriate wound response. Excess deposition and increased crosslinking of the collagen and ECM occurs, resulting in excessive accumulation of ECM above normal requirements, which is associated with persistent myofibroblast activation, damage to epithelial cells and a loss of the normal tissue architecture and function.

Aberrant wound healing (fibrosis) may concern any organ or tissue, for example kidney, lung, intestine, skin, aorta, or liver, originating a variety of fibrotic diseases. The causes of fibrotic diseases may vary according to the organ or tissue involved and remain unknown in many cases. Liver fibrosis and ultimately cirrhosis results from chronic liver damage by exposure to a variety of factors including environmental and dietary factors or infectious agents. Sustained over consumption of alcohol or a high fat/sugar diet may also lead to cirrhosis of the liver. Similarly, diabetes, hypertension, exposure to toxic agents and various types of autoimmune disease may damage the kidneys leading to fibrotic remodeling and loss of kidney function. Many types of inflammatory bowel disease such as Crohn's disease or celiac sprue may lead to fibrotic remodeling causing strictures and/or malabsorption.

Treatment of progressive fibrosis should aim at curing the underlying causal disease. For example, avoid hypertension with a better control of blood pressure, improve glucose management in diabetes, or avoid exposure to the damaging allergen or toxic. However, some fibrotic diseases do not respond adequately to the treatment of the causal injury and organ failure may progress regardless the status of the causal injury eliciting the initiation of the wounding processes. This is the case of chronic kidney disease, in which progression to advanced kidney disease or end stage renal failure may be observed after treatment of the immunological injury, correction of hypertension or improvement of diabetes to cite some of the causal diseases of renal fibrosis.

Much progress has been achieved in the understanding of the mechanisms leading to the different fibrotic diseases. Many different cell types have been related to wound healing, but also to fibrosis and fibrotic disease self-perpetuation. Knowledge has also increased on the role played by the different cellular products that influence fibrosis; many having pro- fibrosis effects and others a protective effect against fibrosis. Among the former CD4+Th2 cell response with IL-4, IL-5, IL-13 and IL-21 production results in an increase in the fibrotic processes, whilst the CD4+Th1 cells with Interferon y and IL-12 chemokines are protective against fibrosis. Transforming growth factor beta (TGF-β) pathway has been demonstrated to participate in nearly all types of fibrosis, although IL-4 has been demonstrated to be much more potent than TGF-β in inducing fibrotic response. Other molecules or genes that have been reported to participate include procollagens I, III and VI, arginase-1, lysyl oxidase, matrix metalloproteinase-2 (MMP-2), MMP-9 and tissue inhibitor of matrix metalloproteinase-1 (TIMP-1) as well as haemoxygenase, procollagen III, secreted phosphoprotein 1, procollagen V, reticulocalbin and fibrillin 1. The pro-fibrotic effects of Angiotensin II have been extensively studied, particularly in heart fibrosis.

Despite the overwhelming progress in the identification of the cells involved and the molecular mechanisms that participate in the switch from the necessary wound healing processes to an abnormal and detrimental self-maintained fibrosis, very few specific treatments can be proposed at the present time for fibrotic diseases.

Pirfenidone is a small molecule drug that was approved for use in the treatment of idiopathic pulmonary fibrosis (IPF) in Japan in 2008 and Europe in 2011 which would have an antifibrotic activity by down-regulating TGF-β. Nintedanib is a tri-angiokinase inhibitor which reduces or blocks VEGF, FGF and PDGF induced tyrosine kinase activity. Although both compounds reduce fibrosis in IPF, their demonstrated clinical benefits are limited (2 years extended survival) and their side effects numerous.

No other treatment has received approval for fibrosis related diseases to the present. Nevertheless, 45% of all deaths in the developed world are attributed to some type of chronic fibroproliferative disease. Therefore, there is an unmet medical need for specific treatments of fibrotic diseases with a very high societal impact. Accordingly, it is an object of the present invention to provide a new method of treatment of fibrosis diseases.

In the present invention, the results disclosed herein demonstrate that specific glycomimetic compounds have anti-fibrosis effects both *in vitro* and *in vivo,* and therefore that said compounds may be used to design effective new methods for the treatment of fibrotic diseases. Any references to methods of treatment in this description are to be interpreted as references to the compounds, pharmaceutical compositions or medicaments of the present invention for use in those methods of treatment.

The international application WO2014128429 discloses a family of heterocyclic phosphonic compounds, in particular compound 3-Hydroxy-4,5-bis-benzyloxy-6-benzyloxymethyl-2-phenyl-2-oxo-2λ5-[1,2]oxaphosphinane for reducing or preventing the appearance of metastases in a patient suffering from cancer.

### SUMMARY OF INVENTION

The present invention provides a family of heterocyclic phosphonic compounds, in particular compound 3-Hydroxy-4,5-bis-benzyloxy-6-benzyloxymethyl-2-phenyl-2-oxo-2λ5-[1,2]oxaphosphinane, for use in the treatment of fibrotic diseases.

### DESCRIPTION OF THE FIGURES

**Figure 1****.** Staining of kidney sections of control and renal failure rat groups. A) Red sirius staining as fibrosis marker. B) Immunostaining of Mgat5. Magnification x200. (*p ≤0.05; **p ≤0.01)
**Figure 2****.** Red sirius staining of kidney sections. C: control + Placebo. C + 3.1 : control + compound 3.1. SNX : SNX + Placebo. SNX + 3.1 : SNX + compound 3.1. Magnification x200. (*p ≤0.05)
**Figure 3****.** Kidney section immunostaining of collagens 1, 3 and 4. A) Collagen 1. B) Collagen 4. C) Collagen 3. C: control + Placebo. C + 3.1 : control + compound 3.1. SNX : SNX + Placebo. SNX + 3.1 : SNX + compound 3.1. Magnification x200. (*p ≤0.05; **p ≤0.01; ***p ≤0.001 et **** p ≤ 0.0001)
**Figure 4****.** L PHA staining as glycans marker of MGAT5 activity on kidney sections. C: control + Placebo. C + 3.1 : control + compound 3.1. SNX SNX + Placebo. SNX + 3.1 SNX + compound 3.1. Magnification x200. (*p ≤0.05; **p ≤0.01)
**Figure 5****.** Staining of aortic ring sections cultured in control and calcifying medium A) Von Kossa staining as calcification marker. B) Red sirius staining C) Immunostaining of GnT-V. Magnification x200.
**Figure 6****.** Cutting pattern of left lateral lobes of mice liver for histological analysis. a and c cuts stored at -80°C. b cuts used for immunostaining.
**Figure 7****.** NASH model mice body weight follow-up during 21 days. Vehicle group: Placebo, 10 ml/kg, per os, twice daily. Compound 3.1 group: 15 mg/kg, per os, twice daily. Telmisartan group: 10 mg/kg, per os, once daily. (*p ≤0.05; **p ≤0.01; ***p ≤0.001 et **** p ≤ 0.0001)
**Figure 8****.** Red sirius staining of mice liver histological cuts. Magnification x200.
**Figure 9****.** Quantification of fibrotic area using Red sirius staining of mice liver histological cuts. Area expressed in percent and corresponding to the ratio: Red sirius red stained surface / cut surface. Magnification x200. (p<0.01)

### DETAILED DESCRIPTION OF THE INVENTION

The present invention relates to the use of heterocyclic phosphonic compounds of formula (1) as detailed below, and in particular compound 3-Hydroxy-4,5-bis-benzyloxy-6-benzyloxymethyl-2-phenyl-2-oxo-2λ5-[1,2]oxaphosphinane (also named as compound 3.1), for treating fibrosis diseases. Said compounds have been previously described as anticancer agents and in particular for reducing or preventing the appearance of metastases, as disclosed in PCT patent applications WO2009/004096 and WO2014/128429.

The compounds used according to the invention have the following formula (1): 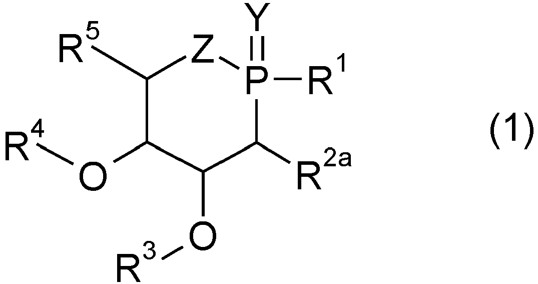
wherein Y represents an oxygen atom,
Z represents O, S, Se, NH or a NR₆ group, wherein R₆ is an aryl or an optionally substituted alkyl group, preferably an oxygen atom,
R₁ represents a hydrogen atom, an optionally substituted alkyl group or an aryl group,
R^{2a} represents a hydrogen atom, halogen, azide (N₃), a carbonate or dithiocarbonate group, a 1H-[1,2,3]triazolyl group or a group -X-R2, wherein
X represents an oxygen, a sulfur, a selenium atom, a NH or NR₇ group, R₇ being an optionally substituted aryl or alkyl group; X preferably represents O or NH, and;
R₂ represents an aryl group, an optionally substituted alkyl group, a hydrogen atom, a trichloroacetimidate group (-C(=NH)CCl₃), an acyl, formyl, sulfonyl, sulfinyl, tert-butyldiphenylsilyl, allyl group, a saccharyl, ester, amide, thioamide, sulfonamide group, or X-R₂ represents a P(O)R₂R₆ group, in which R₂ and R₆ represent independently from each other an aryl group, an optionally substituted alkyl group, OH, an alkoxy or an aryloxy group, R₃ and R₄ represent independently from each other an aryl, an optionally substituted alkyl group, an hydrogen atom, a trichloroacetimidate group, an acyl, formyl, sulfonyl, sulfinyl, tert-butyldiphenylsilyl group, an allyl, a saccharyl, ester, amide, thioamide, sulfonamide group, or R₃ and R₄ taken together form a divalent radical of formula -R₃-R₄-, wherein -R₃-R₄- preferably represents an isopropylidene, benzylidene, diphenyl methylidene, cyclohexyl methylidene group, and their substituted analogues, for example a 4-methoxybenzylidene group, or a linear alkylene group such as an ethylene group (so as to form a propane-1,2-diol group),
R₅ represents a hydrogen atom or a hydrocarbon group comprising one or more heteroatoms, preferably selected from oxygen, sulfur or nitrogen, more preferably oxygen.

In the present description of chemical compounds, the names are typically employed according to their usual definition.

As used herein, "alkyl" means a linear or branched, saturated or unsaturated hydrocarbon group, having from 1 to 25 carbon atoms, including in particular the acyclic groups with from 1 to 8 carbon atoms, such as methyl, ethyl, n-propyl, isopropyl, butyl, n-hexyl groups; cycloalkyl groups having preferably from 3 to 7 carbon atoms, cycloalkylmethyl groups having preferably from 4 to 8 carbon atoms.

As used herein, "substituted alkyl" means an alkyl group such as defined hereabove, that is bound through a sp3 carbon atom and substituted with one or more aryl groups and/or comprising one or more heteroatoms such as N, S or O. Suitable examples include arylalkyl groups such as (-CPh3)-trityl group, benzyl group (noted Bn) or 4-methoxybenzyl group, alkoxyalkyl groups, especially dialkoxymethyl groups such as diethoxymethyl or dimethoxymethyl groups, CH2CO2R11 groups, wherein R11 represents an optionally substituted alkyl group or an aryl group.

As used herein, "alkoxy" means an alkyl group that is bound to the rest of the molecule through an oxygen atom, for example an ethoxy, methoxy, or n-propoxy group.

As used herein, "aryloxy" means an aryl group bound to the rest of the molecule through an oxygen atom, for example a benzoxy group.

As used herein, "acyl" means a group derived from a carboxylic acid by removing the hydroxyl group, having preferably the formula -C(O)R8, wherein R8 represents an aryl or an optionally substituted alkyl group, for example an acetyl, trifluoro acetyl, propionyl, oleoyl, myristoyl or benzoyl group.

As used herein, "sulfonyl" means a group derived from a sulfonic acid by removing the hydroxyl group, having preferably the formula -SO2R9, wherein R9 represents an optionally substituted alkyl group or an aryl group.

As used herein, "sulfinyl" means a radical derived from a sulfinic acid by removing the hydroxyl group, having preferably the formula -SOR10, wherein R10 represents an optionally substituted alkyl group or an aryl group.

As used herein, "dithiocarbonate group" means a group of formula -OC(S)SR9c, wherein R9c represents an optionally substituted alkyl group or an aryl group.

As used herein, "carbonate group" means a group of formula -OC(O)OR9d, wherein R9d represents an optionally substituted alkyl group or an aryl group.

As used herein, an "ester group" means a group of formula -C(O)OR10', wherein R10' represents an optionally substituted alkyl group or an aryl group.

As used herein, an "amide group" means a group of formula -C(O)NR9'R9", wherein R9' represents an optionally substituted alkyl group or an aryl group and R9" represents an optionally substituted alkyl group, an aryl group or a hydrogen atom.

As used herein, a "thioamide group" means a group of formula -C(S)NR9aR9b, wherein R9a represents an optionally substituted alkyl group or an aryl group and R9b represents an optionally substituted alkyl group, an aryl group or a hydrogen atom.

As used herein, a "sulfonamide group" means a group of formula -SO2NR11'R11", wherein R11' represents an optionally substituted alkyl group or an aryl group and R11" represents an optionally substituted alkyl group, an aryl group or a hydrogen atom.

As used herein, "aryl" means an aromatic monovalent carbocyclic radical comprising only one ring (for example a phenyl group) or a plurality of fused rings (for example the naphthyl and terphenyl groups), which may optionally be substituted with one or more groups such as, without limitation, the alkyl (for example methyl), hydroxyalkyl, amino-alkyl, hydroxyl, thiol, amino, halogeno (fluoro, bromo, iodo, chloro), nitro, alkylthio, alkoxy (for example methoxy), aryloxy, mono-alkylamino, dialkylamino, acyl, carboxyl, alkoxycarbonyl, aryloxycarbonyl, hydroxysulfonyl, alkoxysulfonyl, aryloxysulfonyl, alkylsulfonyl, alkylsulfinyl, cyano, trifluoromethyl, tetrazolyl, carbamoyl, alkylcarbamoyl and dialkylcarbamoyl groups. Alternatively, two adjacent positions in the aromatic ring may be substituted with a methylenedioxy or ethylenedioxy group. As used herein, "aryl" also includes the "heteroaryl" groups, that is to say the aromatic rings wherein one or more carbon atoms of the one or more aromatic rings are substituted with one heteroatom such as a nitrogen, oxygen, phosphorus or sulfur atom. The heteroaryl groups may be one or several aromatic rings-containing structures or structures with only one or several aromatic rings coupled to one or more non aromatic rings. In structures possessing many rings, the rings may be fused, covalently bound or bound to each other through a divalent common group such as a methylene, ethylene or a carbonyl group. Suitable examples of heteroaryl groups include the thiophene groups (2-thienyl, 3-thienyl), pyridine groups (2-pyridyl, 3-pyridyl, 4-pyridyl), isoxazole, phthalimide, pyrazole, indole and furan groups, as well as their benzofused analogues, phenyl pyridyl ketone, quinoline, phenothiazine, carbazole and benzopyranone.

As used herein, a "saccharyl group" includes all radicals derived by removing a hydroxyl group or a hydrogen atom (preferably a hydroxyl group), from a natural or synthetic, protected or unprotected carbohydrate or sugar. The saccharyl group can include the monosaccharyl or oligosaccharyl groups, such as disaccharyl groups. The saccharyl groups, for example glucosyl and mannosyl groups may be derived from sugars such as, without limitation, the glucuronic acid, the lactose, the sucrose, the maltose, the allose, the alltrose, the glucose, the mannose, the idose, the galactose, the talose, the ribose, the arabinose, the xylose, the lyxose, the fructose, the threose, the erythrose, the [beta]-D-N-acetylgalactosamine, the [beta]-D-N- acetylglucosamine, the fucose, the sialic acid, the N-acetylneuraminic acid, the N-acetylmuramic acid, the glucosamine, the galactosamine, the rhamnose and their protected or substituted analogues, that are substituted for example with acyl, alkyl, aryl, halogeno and amino groups, as well as their desoxy type analogues. As used herein, an oligosaccharyl group means a saccharyl group derived from at least two covalently bound monosaccharides, comprising preferably from 1 to 3 saccharide units. For a description of saccharide type structures, see "Essentials of Glycobiology," Varki and al. Eds., Chapter 2 (Cold Spring Harbor Press, Cold Spring Harbor, N.Y., 1999). Preferred saccharyl groups are monosaccharyl groups. In compounds of formula (1 ), when R2a represents -X-R2 group, wherein R2 represents a saccharyl group, said saccharyl group is preferably bound through a X group representing O or NH, preferably O.

As used herein, a "saccharide" means a monosaccharide or an oligosaccharide.

"Bn" stands for a benzyl group, "Ac" an acetyl group.

Some compounds of the invention may equally present in a solvated or a non-solvated form, for example as an hydrate. Generally, solvated forms are equivalent to non-solvated forms and are included within the frame of the invention. Some compounds of the invention may have a plurality of various crystalline or amorphous forms. Generally, all physical forms are equivalent for the uses that are intended according to the present invention and are included within the frame of the present invention.

The compounds of the invention have several asymmetric (optical) centers, so that enantiomers or diasteroisomers may exist. It is understood that the present invention does include all the enantiomers and diasteroisomers of the compounds of formula (1 ), as well as their mixtures, especially those based on racemates. The different isomers may be separated according to methods known to those skilled in the art, notably silica gel chromatography- or fractional crystallisation-based methods.

The preferred compounds of formula (1) are those wherein Y = Z = O, that is to say 1 ,2-oxaphosphinane 2-oxide compounds.

In the compounds of the invention, R₁ substituent, where it does not represent a hydrogen atom, is always bound to the intracyclic phosphorus atom through a carbon atom.

Preferred R₁ groups include H, alkyl groups, such as 2-benzyloxyethyl, ethyl, n-butyl, 3-phenylpropyl, n-octyl, dialkoxymethyl groups such as a diethoxymethyl or dimethoxymethyl group, aryl groups, such as phenyl, 4-methylphenyl, 4-nitrophenyl, 4-aminophenyl, 4-methoxyphenyl, 3,4-difluorophenyl, 2-thienyl, 4-fluorophenyl, 4-biphenyl, 3-methylphenyl, 3- methoxyphenyl and 3,5-difluorophenyl groups, as well as the following groups:

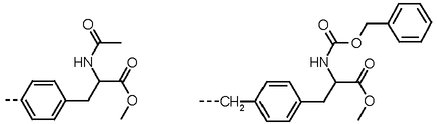

In a particular embodiment, R1 is a phenyl group.

Preferred R₂ groups include H, arylsulfonyl, methylsulfonyl, trichloroacetimidate, benzyl, saccharyl and aryl groups, such as phenyl, 4-methylphenyl, 4-nitrophenyl, 4-aminophenyl, 3,4- difluorophenyl, 3,5-difluorophenyl, and 3,4-dinitrophenyl groups.

Preferred X-R2 groups include O-aryl, OH, NH2, NH-aryl, S-aryl and dithiocarbonate groups, or NHCH2CO2R11, wherein R11 is such as defined hereabove, NHC(O)R12, wherein R12 represents an aryl group or an optionally substituted alkyl group, O-SO2R9 wherein R9 is such as defined hereabove, NH-Bn, O-saccharyl, OC(=NH)CCl3, phosphonic acid, phosphinic acid or phosphine oxide, urea, thiourea, carbamate and carbonate groups. According to a particular embodiment, X-R2 is OH, and preferably R1 is a phenyl group. Preferably, R3 and R4 represent independently from each other, a hydrogen atom, a benzyl, benzoyl or an acetyl group, or they form together a divalent radical of formula -R3-R4-representing preferably an isopropylidene group.

According to a particular embodiment, R3 and R4 represent a benzyl group and/or R1 is a phenyl group and/or X-R2 is OH.

According to another particular embodiment, R3 and R4 represent a benzyl group and preferably R1 is a phenyl group and/or X-R2 is OH.

According to a preferred embodiment of the invention, R₅ is such that the compounds of formula (1) have the following formula (2) or (3): 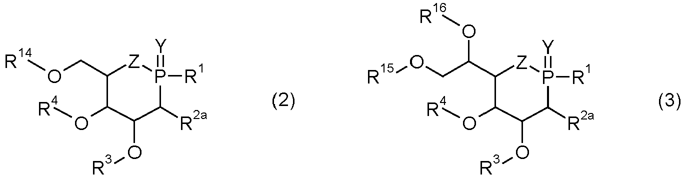 wherein R1, R^{2a}, R3, R4, Y and Z are as defined hereabove, R14, R15 and R16 represent, independently from each other, a hydrogen atom, an aryl, an optionally substituted alkyl group, a trichloroacetimidate group, an acyl, formyl, sulfonyl, sulfinyl, tert-butyldiphenylsilyl group, an allyl, ester, amide, thioamide, sulfonamide group, or R15and R16, taken together, form a divalent radical of formula -R¹⁵-R¹⁶-, wherein -R¹⁵-R¹⁶- preferably represents an isopropylidene, benzylidene, diphenyl methylidene, cyclohexyl methylidene group, and their substituted analogues, for example a 4-methoxybenzylidene group, or a linear alkylene group such as an ethylene group.

According to a particular embodiment, R14 represents a benzyl group, and preferably with at least one or more particular embodiments as above detailed, including where R3 and R4 represent a benzyl group and/or R1 is a phenyl group and/or X-R2 is OH.

R₅ when not representing a hydrogen atom, does preferably have from 1 to 25 carbon atoms, preferably from 1 to 20 carbon atoms, more preferably from 1 to 10 carbon atoms, and even more preferably from 1 to 8 carbon atoms. R₅ may represent an optionally substituted alkyl group comprising one or more heteroatoms preferably selected from oxygen, sulfur or nitrogen, more preferably oxygen. Preferred R₅ groups include alkoxyalkyl groups such as benzyloxymethyl (-CH₂OBn), -CH₂OH, 2,2-dimethyl-[1,3]-dioxolan-4-yl and 1,2-dihydroxy-ethyl CH(OH)CH₂OH groups, which means in the formulas (2) and (3) that R¹⁴ = H or Bn, and R¹⁵ = R¹⁶ = H or R¹⁵ and R¹⁶, taken together, do form an isopropylidene radical.

According to a particular embodiment, the compound useful in the treatment of fibrosis is selected in the group consisting of
3-Hydroxy-4,5-bis-benzyloxy-6-benzyloxymethyl-2-phenyl-2-oxo-2λ5-[1,2]oxaphosphinane,
4-(2,2-Dimethyl-[1,3]dioxolan-4-yl)-2,2-dimethyl-2-oxo-2-phenyl-tetrahydro-6 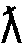 *5*-[1,3]dioxolo[4,5-d][1,2]oxaphosphinan-3-aminobenzyl, more specifically (3aR,6S,7S,7aS)-7-(benzylamino)-4-((R)-2,2-dimethyl-1,3-dioxolan-4-yl)-2,2-dimethyl-6-phenyltetrahydro-[1,3]dioxolo[4,5-d][1,2]oxaphosphinine 6-oxide (also named herein compound 3.3),
N-((2S,3S,4S,5S,6R)-4,5-bis(benzyloxy)-6-((benzyloxy)methyl)-2-oxido-2-phenyl-1,2-oxaphosphinan-3-yl)acetamide (also named herein compound 2.2),
4,5-bis-benzyloxy-6-benzyloxymethyl-phenyl-2-oxo-2 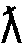 5-[1,2]oxaphosphinan-3-aminobenzyl, more specifically (2S,3S,4S,5S,6R)-3-(benzylamino)-4,5-bis(benzyloxy)-6-((benzyloxy)methyl)-2-phenyl-1,2-oxaphosphinane (also named herein compound 4.6),,
(2S,3S,4S,5S,6R)-4,5-bis(benzyloxy)-6-((benzyloxy)methyl)-3-hydroxy-2-(4-phenoxyphenyl)-1,2-oxaphosphinane 2-oxide (also named herein compound 3.0), and
(3aR,6R,7R,7aS)-4-((R)-2,2-dimethyl-1,3-dioxolan-4-yl)-2,2-dimethyl-6-oxido-6-phenyltetrahydro-[1,3]dioxolo[4,5-d][1,2]oxaphosphinin-7-yl benzoate (also named herein compound 4.2).

In a more particular embodiment, the compound useful in the treatment of fibrosis is 3-Hydroxy-4,5-bis-benzyloxy-6-benzyloxymethyl-2-phenyl-2-oxo-2λ5-[1,2]oxaphosphinane. Preparation of compound 3-Hydroxy-4,5-bis-benzyloxy-6-benzyloxymethyl-2-phenyl-2-oxo-2λ5-[1,2]oxaphosphinane can for example be carried out as described in WO 2009/004096, WO 2014/128429, and WO 2018/054925.

The compound 3-Hydroxy-4,5-bis-benzyloxy-6-benzyloxymethyl-2-phenyl-2-oxo-2λ5-[1,2]oxaphosphinane to be used according to the invention has preferably the following Formula (I):

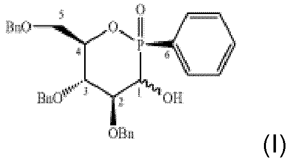

Accordingly, the invention relates to a compound of formula (1), preferably 3-Hydroxy-4,5-bis-benzyloxy-6-benzyloxymethyl-2-phenyl-2-oxo-2λ5-[1,2]oxaphosphinane, and more preferably of formula (I), also called PST3.1, for use in a method for the treatment of fibrotic diseases.

In a further aspect, the invention relates to the compound of formula (1) as detailed above for use in the treatment of fibrosis by inhibition of GnT-V activity.

In a further aspect, the invention relates to the compound of Formula (1) for use in the treatment of fibrosis by inhibition of the production of collagen fibers, more specifically collagen of types 1, 3 and/or 4, and/or by inhibition of mechanisms implicated in cell matrix and/or cell/cell interactions, including inhibition of fibroblast migration.

The invention further provides for a use of a compound of formula (1) as defined herein, in particular 3-Hydroxy-4,5-bis-benzyloxy-6-benzyloxymethyl-2-phenyl-2-oxo-2λ5-[1,2]oxaphosphinane, and preferably of formula (I), also called PST3.1, for the manufacture of a medicament or pharmaceutical composition for the treatment of fibrotic diseases. In a particular aspect, the treatment of fibrosis is by inhibition of GnT-V activity. In a further particular aspect, the treatment of fibrosis is by inhibition of the production of collagen fibers, more specifically collagen of types 1, 3 and/or 4, and/or by inhibition of mechanisms implicated in cell matrix and/or cell/cell interactions, including inhibition of fibroblast migration.

The invention further provides for a method for treating fibrotic diseases in a patient in need thereof by administering in a patient in need of such treatment an effective amount of a compound of formula (1) as defined herein, in particular 3-Hydroxy-4,5-bis-benzyloxy-6-benzyloxymethyl-2-phenyl-2-oxo-2λ5-[1,2]oxaphosphinane, and preferably of formula (I), also called PST3.1. In a particular aspect, the treatment of fibrosis is by inhibition of GnT-V activity. In a further particular aspect, the treatment of fibrosis is by inhibition of the production of collagen fibers, more specifically collagen of types 1, 3 and/or 4, and/or by inhibition of mechanisms implicated in cell matrix and/or cell/cell interactions, including inhibition of fibroblast migration.

According to the present invention, the term "fibrosis" includes in particular a lung, kidney, liver, heart, muscle, skin, soft tissue (e.g. mediastinum or retroperitoneum), bone marrow, intestinal, aortic and joint (e.g. knee, shoulder or other joints) fibrosis. In particular, the term "fibrotic disease" resulting from fibrosis includes, pulmonary fibrosis, idiopathic pulmonary fibrosis, cystic fibrosis, endomyocardial fibrosis, mediastinal fibrosis, myelofibrosis, retroperitoneal fibrosis, progressive massive fibrosis (a complication of coal workers' pneumoconiosis), nephrogenic systemic fibrosis, Crohn's disease, keloid, old myocardial infarction, scleroderma, systemic sclerosis, arthrofibrosis and some forms of adhesive capsulitis.

According to a particular embodiment, the compound of formula (1) is for use in the treatment of liver, kidney or skin fibrosis, more specifically in the treatment of kidney or skin fibrosis, including keloids or scleroderma.

According to a particular embodiment, the invention relates to the use of a compound of formula (1) as defined herein, for the manufacture of a medicament or pharmaceutical composition for the treatment of liver, kidney or skin fibrosis, more specifically in the treatment of kidney or skin fibrosis, including keloids or scleroderma.

According to a particular embodiment, the invention further relates to a method for treating liver, kidney or skin fibrosis, more specifically for treating kidney or skin fibrosis, including keloids or scleroderma, in a patient in need thereof, by administering in a patient in need of such treatment an effective amount of a compound of formula (1) as defined herein.

According to another particular embodiment, the compound of formula (1) is for use in the treatment of aortic fibrosis.

According to a particular embodiment, the invention relates to the use of a compound of formula (1) as defined herein, for the manufacture of a medicament or pharmaceutical composition for the treatment of aortic fibrosis.

According to a particular embodiment, the invention further relates to a method for treating aortic fibrosis in a patient in need thereof, by administering in a patient in need of such treatment an effective amount of a compound of formula (1) as defined herein.

A compound of formula (1) may be provided in a pharmaceutical composition. The pharmaceutical composition may additionally comprise a pharmaceutically acceptable adjuvant and/or carrier.

As used herein, "pharmaceutically acceptable carrier" includes any and all solvents, dispersion media, coatings, antibacterial and antifungal agents, isotonic and absorption delaying agents, and the like that are physiologically compatible. The carrier may be suitable for parenteral, e.g. intravenous, intramuscular, intradermal, intraocular, intraperitoneal, subcutaneous, spinal or other parenteral routes of administration, for example by injection or infusion. Alternatively, the carrier may be suitable for non-parenteral administration, such as a topical, epidermal or mucosal route of administration. The carrier may be suitable for oral administration. Depending on the route of administration, the compound of the invention may be coated in a material to protect it from the action of acids and other natural conditions that may inactivate it. The pharmaceutical compositions of the invention may include one or more pharmaceutically acceptable salts. A "pharmaceutically acceptable salt" refers to a salt that retains the desired biological activity of the parent compound and does not impart any undesired toxicological effects. Examples of such salts include acid addition salts and base addition salts.

Pharmaceutically acceptable carriers comprise aqueous carriers or diluents. Examples of suitable aqueous carriers that may be employed in the pharmaceutical compositions of the invention include water, buffered water and saline. Examples of other carriers include ethanol, polyols (such as glycerol, propylene glycol, polyethylene glycol, and the like), and suitable mixtures thereof, vegetable oils, such as olive oil, and injectable organic esters, such as ethyl oleate. In many cases, it will be desirable to include isotonic agents, for example, sugars, polyalcohols such as mannitol, sorbitol, or sodium chloride in the composition. Therapeutic compositions typically must be sterile and stable under the conditions of manufacture and storage. The composition can be formulated as a solution, microemulsion, liposome, or other ordered structure suitable to high drug concentration.

Pharmaceutical compositions of the invention may comprise additional active ingredients. Also within the scope of the present invention are kits comprising a compound of formula (1) as defined herein and instructions for use, in particular for the treatment of fibrosis diseases. The kit may further contain one or more additional reagents, such as an additional therapeutic or prophylactic agent as discussed above. The compound of the invention or the composition comprising the same may be administered for the treatment of a fibrotic disease.

In one embodiment the treatment of a fibrotic disease is a therapeutic treatment. In therapeutic applications, compounds are administered to a subject already suffering from a disorder or condition as described above, in an amount sufficient to cure, alleviate or partially arrest the condition or one or more of its symptoms. Such therapeutic treatment may result in a decrease in severity of disease symptoms, or an increase in frequency or duration of symptom-free periods. An amount adequate to accomplish this is defined as a "therapeutically effective amount".

In one embodiment the treatment of a fibrotic disease is a prophylactic treatment. In prophylactic applications, formulations are administered to a subject at risk of a disorder or condition as described above, in an amount sufficient to prevent or reduce the subsequent effects of the condition or one or more of its symptoms. An amount adequate to accomplish this is defined as a "prophylactically effective amount".

The treatment involves the administration of the compound or a pharmaceutical composition containing the same to a patient having a declared disorder to cure, delay, or slow down the progress, thus improving the condition of the patient or to a healthy subject, in particular a subject who is at risk of developing a fibrotic disease.

The subjects to be treated according to the invention can be selected on the basis of several criteria associated to fibrotic diseases such as previous drug treatments, associated pathologies, genotype, exposure to risk factors, viral infection, as well as any other relevant biomarker that can be evaluated by means of imaging methods and immunological, biochemical, enzymatic, chemical, or nucleic acid detection method.

Effective amounts for each purpose will depend on the severity of the disease or injury as well as the weight and general state of the subject.

A subject for administration may be a human or non-human animal. The term "non- human animal" includes all vertebrates, e.g., mammals and non-mammals, such as non- human primates, sheep, dogs, cats, horses, cows, chickens, amphibians, reptiles, etc. Administration to humans is typical.

The compound of Formula (1) may be administered in an effective amount by using a pharmaceutical composition as above-defined. Within the context of the invention, the term "effective amount" refers to an amount of the compound sufficient to produce the desired therapeutic result.

The frequency and/or dose relative to the administration can be adapted by one of ordinary skill in the art, in function of the patient, his body weight, the pathology, the form of administration, etc. Administration can be performed daily or even several times per day, if necessary.

In a further embodiment, the present invention provides methods of treating fibrotic diseases comprising the administration to a subject in need of such treatment of an effective amount of at least one compound of Formula (1) or a pharmaceutical composition containing the same.

The invention is further described with reference to the following, non-limiting, examples.

### EXAMPLES

### Example 1

### Materials & Methods

Male Sprague-Dawley rats underwent 5/6 subtotal nephrectomy (SNx) and were used as the kidney fibrosis model, since SNx rats developed tubulointerstitial fibrosis. During this surgery, the right kidney is removed and 2 of the 3 branches of the left renal artery are ligated in order to necrose 2/3 of the kidney. The control kidney is right kidney before nephrectomy. Tubulointerstitial renal fibrosis inevitably leads to renal function deterioration and failure, independently of the primary renal disease. It is worth noting that in SNx rats, plasmatic creatinine concentration increases from 25.7±1.3µM to 120.0±33.0 µM. Sirius red staining highlights fibrosis by staining collagens in paraffin-embedded kidney tissue sections. Five micrometer-thick sections were cut and mounted on glass slides. Slides were deparaffinized and stained with Sirius red for evaluation of collagen localization. Positive signal for collagen was seen in intense red. The sections were mounted in Entelan mounting medium and examined under microscope. For each sample, quantification was performed with Image J software on pictures taken from 10 fields of kidney sections at 200-fold magnification. The staining area was measured on kidney section fields. Immunohistochemical analyses of kidney sections were also performed using paraffin sections. The primary antibody, anti-Mgat5 was incubated overnight at 4°C. Revelation was performed with Universal vectastain ABC kit and ImmPACT AE following the instructions of suppliers, Vector Laboratories. Then, the sections were mounted in an aqueous mounting medium, VectaMount ^{™} AQ and examined under microscope (Nikon Eclipse TE300). Quantification was carried out as for histological staining.

### Results

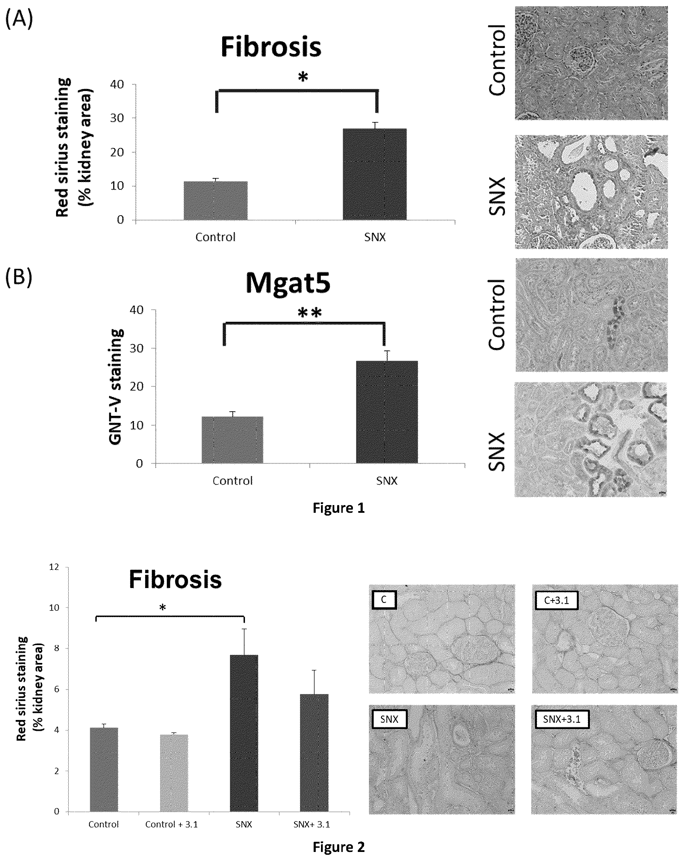

It is demonstrated in a subtotal nephrectomy (SNx) model the development of fibrosis and Mgat5 overexpression in 12-week-old rats with renal failure (Figure 1). Before the nephrectomy, the collagen fibers are distributed evenly; 12 weeks after surgery, numerous aggregates of fibrosis are observed. The results show a significant increase of 15.4 ± 1.4% in fibrosis. The expression of Mgat5 is also significantly increased after surgery by 14.5 ± 2.6%. These results suggest that increased renal fibrosis is associated with increased expression of Mgat5.

### Example 2

### Materials & Methods

Compound 3.1 was orally administered to control rats and SNx rats (as above described) at 20mg/kg daily per 28 days. A control was carried out with a vehicle (without compound 3.1) administered in the same manner as compound 3.1. Sprague Dawley rats will be used. The rats will be fed ad libitum and will be placed in a 12 hour light, 12 hour dark cycle. They will be divided 3 per cage. Some rats undergo 5/6 subtotal nephrectomy (SNx) to develop chronic kidney failure which will lead to the development of renal fibrosis. The 3.1a treatment was administered to the rats on the day of nephrectomy and continued for the entire protocol. It is delivered as a nanosuspension diluted in drinking water. Control rats receive the solvent alone (placebo). Histological and immunohistochemical methods were monitored as described in example 1.

### Results

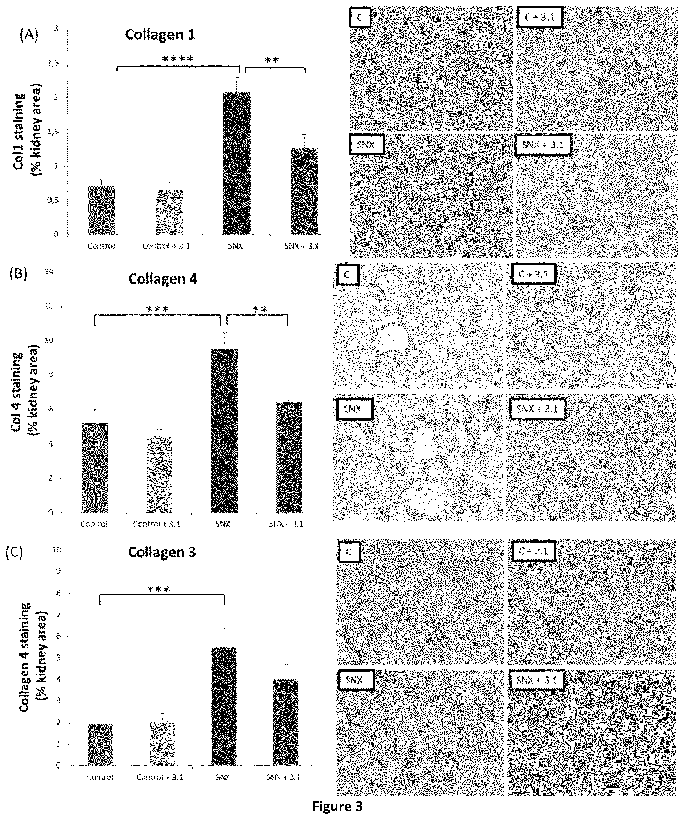

At 4 weeks, plasma creatinine concentration was measured in control and SNx rats taking 3.1 and placebo. Creatinine increased significantly in nephrectomized rats (p <0.0001) which validates the model. SNx treated rats have lower serum creatinine than SNx rats. The development of fibrosis can be observed by staining with Sirius red on histological sections (Figure 2). At 4 weeks, a significant increased fibrosis was observed (4.1 ± 0.2%) in SNx rats compared to control rats (7.7 ± 1.3%). A decreasing trend of fibrosis with treatment is observed in SNx rats (5.8 ± 1.2%) compared to control SNx. There is no significant effect of treatment on fibrosis in health animals or SNx at this time of observation. Other markers can measure the development of renal fibrosis, such as collagens (Figure 3). The results show a high concentration of collagen I, III and IV in SNx rats compared to control rats. Concerning collagen I, an effect is observed on SNx, the percentage of collagen I tripled after nephrectomy, it goes from 0.7 ± 0.1% for the control group to 2.1 ± 0.2% for the SNx group (p = 0.0001) (Figure 3A). A significant decrease (p = 0.014) in collagen I expression was observed in treated rats with renal impairment (1.3 ± 0.3%) compared to untreated. 3.1 significantly decreases the expression of collagen I in renal fibrosis. The results obtained for the expression of collagen IV are similar to those obtained for collagens I (Figure 3B). The expression of IV collagen went from 5.2 ± 0.8% for the control group to 9.4 ± 1.0% for the SNx group (p = 0.0002). 3.1 significantly decreases IV collagen in renal fibrosis (9.4 ± 1.0% against 6.4 ± 0.3%; p = 0.015). For collagen III, there is also an SNx effect, the percentage of triple collagen III in rats having undergone nephrectomy compared to control rats (5.5 ± 1% vs. 1.7 ± 0.2%, respectively p = 0.0007) (Figure 3C). Treatment with 3.1 shows a tendency to reduce the level of collagen III in the SNx rats (4.0 ± 0.3%) in comparison with the untreated (p = 0.27). In controls, there was no treatment effect on collagen III.

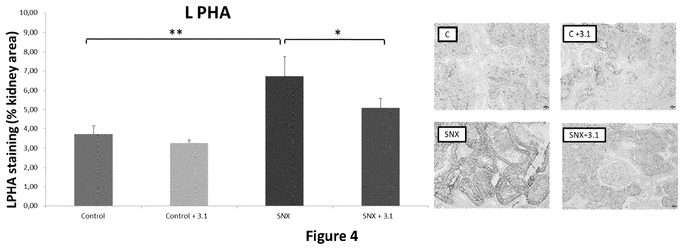

Glycosylation resulting from MGAT5 activity will be specifically measured by L-PHA lectin staining. These results will make it possible to evaluate the effectiveness of the 3.1 treatment on this route (Figure 4). PHA-L staining goes from 3.7 ± 0.4% for the control group to 6.8 ± 1.0% for the SNx group. A significant decrease in PHA-L staining was observed in treated rats with renal impairment (6.8 ± 1.0% against 5.1 ± 0.5 %) compared to untreated.

### Example 3:

### Materials & Methods

A model of vessel fibrosis is *ex vivo* aortic ring calcification. Aortic calcification is a classical complication of renal failure (Chronic Kidney Disease) and is also a model of vascular fibrosis strongly implicated in cardiovascular diseases. The thoracic aortas were harvested from the descending part of the aortic cross to the diaphragm. The adjacent connective tissue was gently removed and the aorta was submitted to three successive PBS washings. Aorta was cut in rings of around 2mm thickness and aortic rings were cultured 14 days in 24 well plates with the basal medium is the Dulbecco's modified Eagle's medium containing 4.5 g.L-1 glucose, 10 mM sodium pyruvate and 50 mg.mL-1 ascorbic acid supplemented with 15% FCS and 3.8 mM NaH2PO4 / Na2HPO4 to induce calcification. Histological and immunohistochemical methods were monitored as described in example 1.

### Results

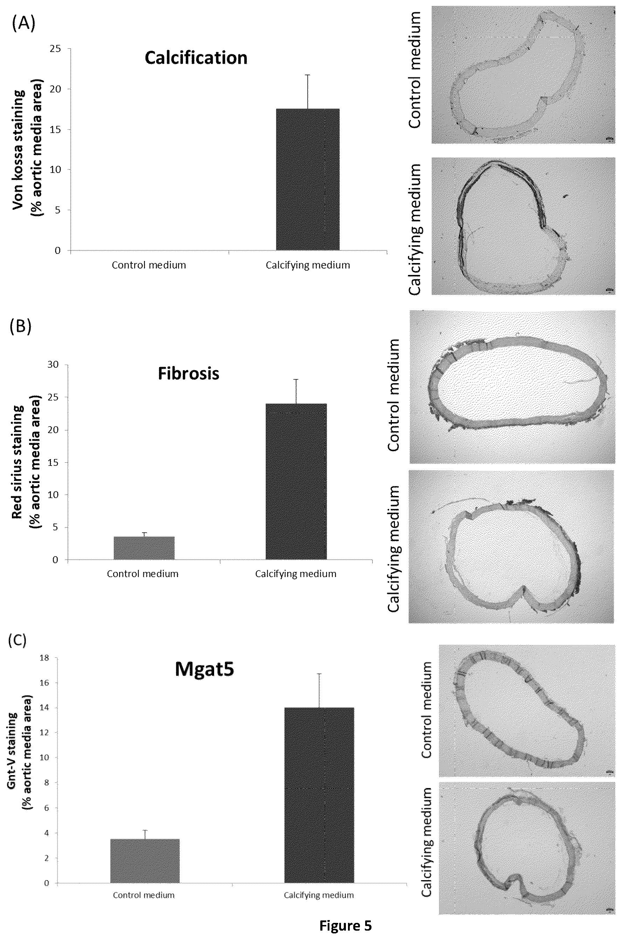

Aortic rings, cultured in calcifying medium displayed a positive von Kossa staining distributed alongside the medial layer of the arterial explant demonstrating mediacalcosis associated with fibrosis (Fig 5A and 5B). No calcium deposits were observed in the aortic rings in control medium. Fibrosis is 5 times higher in the ex vivo model of calcified aortic rings and immunostaining of GnT-V shows a high increase of correspondingGnT-V protein (14.0 ± 2.7% against 3.5 ± 0.7 %) compared to uncalcified aortic rings.

### Example 4:

### Materials & Methods

NIH3T3 fibroblasts were seeded at 40 000 cells per cm2 in a 24 well plate, DMEM + 10% FBS.

Before wound/treatment, cells were washed twice in DMEM without serum then incubated in the appropriate condition DMEM + 2.5% FBS, TGFβ +/-, PST3.1 +/-.

TGFβ 5ng/ml; PST3.1 1µM, n=6 wells per treatment.

At t0, cell layer is scratched using a needle and observed under microscope to take 3 pictures per well using a scale measure referential to measure width of the scratch using imageJ software.

After 24h of incubation in the desired condition, scratch is measured under microscope by taking 3 pictures at the same scale using the same referential.

Data are treated by calculating percentage of closing.

### Results

The results are gathered in Table 1 below.

### % inhibition of wound healing at 1µM in presence of TGFβ

### 24h - 1µM tested compound

**Table 1**

| **Compound (Cpd)** | **% inhibition** |
|---|---|
| **3.3** | 29.8 |
| **2.2** | 28.7 |
| **4.6** | 29.3 |
| **3.0** | 23.0 |
| **4.2** | 17.2 |
| **3.1** | 42.0 |

Compound 3.1 appears the most active compound in this wound healing inhibition test.

### Example 5:

The STAM model (developed and standardized by SMC Laboratories, Japan https://www.smccro-lab.com/) is a model that recapitulates the same disease progression as human nonalcoholic steatohepatitis (NASH) and hepatocellular carcinoma (HCC): in 25% of affected patients (i.e. 20%-25% of the adult population), nonalcoholic fatty liver disease progresses to NASH, which increases the risk for the development of cirrhosis, liver failure, and hepatocellular carcinoma. In patients with NASH, liver fibrosis is the main determinant of mortality (https://doi.org/10.1053/j.gastro.2019.11.311). In this model, male C57BL/6 mice aged two days are given a single dose of streptozotocin to reduce insulin secretory capacity. When the mice turn four weeks of age they start a high-fat diet feeding. This model has a background of late type 2 diabetes which progresses into fatty liver, NASH, fibrosis and consequently liver cancer (HCC). Compared to other NASH-HCC model mice, the disease progresses in a relatively short period of time, and liver cancer is developed in 100% of animals at 20 weeks of age.

The model is able to reproduce many of the pathological features of human NASH:
- ballooning degeneration of cells, a characteristic pathological feature of human NASH; burned-out NASH, in which lipid droplets decrease as fibrosis progresses.
- Progression of fibrosis occuring around the central vein.
- A mild rise in ALT (a liver injury marker).
- Increase in NASH markers such as CK-18.
- Increase in human HCC markers such as: glutamine synthase, glypican-3 and AFP have been observed.

### Materials & Methods

C57BL/6JJmsSIc mice (14-day-pregnant female) were obtained from Japan SLC, Inc. (Japan). Two days after birth, NASH is induced in male mice by a single subcutaneous injection of 200 µg streptozotocin (STZ, Sigma-Aldrich, USA) solution and feeding with high fat diet (HFD, 57 kcal% fat, Cat# HFD32, CLEA Japan, Inc., Japan) after 4 weeks of age. Sterilized solid HFD is provided *ad libitum,* and replaced once every 2 days according to the manufacturer's instructions. Then, NASH model mice are divided into 3 randomized groups of 10 mice at 6 weeks of age based on their body weight the day before the start of treatment. The randomization was performed by body weight-stratified random sampling using Excel software. NASH model mice were stratified by their body weight to get SD and the difference in the mean weights among groups as small as possible.

Group 1: Vehicle (10 NASH mice orally administered with Placebo nanosuspension in a volume of 10 mL/kg twice daily from 6 to 9 weeks of age).

Group 2: Compound 3.1 (PST3.1) ( (10 NASH mice orally administered with compound 3.1 nanosuspension at a dose of 15 mg/kg in a volume of 10 mL/kg twice daily (2*15mg/kg/day) from 6 to 9 weeks of age).

Group 3: Telmisartan (10 NASH mice will be orally administered vehicle supplemented with Telmisartan at a dose of 10 mg/kg once daily in a volume of 10 mL/kg from 6 to 9 weeks of age). Vehicle and compound 3.1 were provided by Phost'in Therapeutics SAS. Telmisartan (Micardis^{®}) was purchased from Boehringer Ingelheim GmbH (Germany).

Telmisartan, a small-molecule antihypertensive drug, has been used for its antifibrotic activity, but its clinical use is limited because it causes systemic hypotension (https://doi.org/10.1038/s41551-018-0279-x).

**Table 2**

| Group | No. mice | Mice | Test substance | Dose (mg/kg) | Volume (mL/kg) | Regimen | Sacrifice |
|---|---|---|---|---|---|---|---|
| 1 | 10 | STAM | Vehicle | - | 10 | PO, BID, 6 - 9 wks | 9 wks |
| 2 | 10 | STAM | Compound 3.1 | 15 | 10 | PO, BID, 6 - 9 wks | 9 wks |
| 3 | 10 | STAM | Telmisartan | 10 | 10 | PO, QD, 6 - 9 wks | 9 wks |

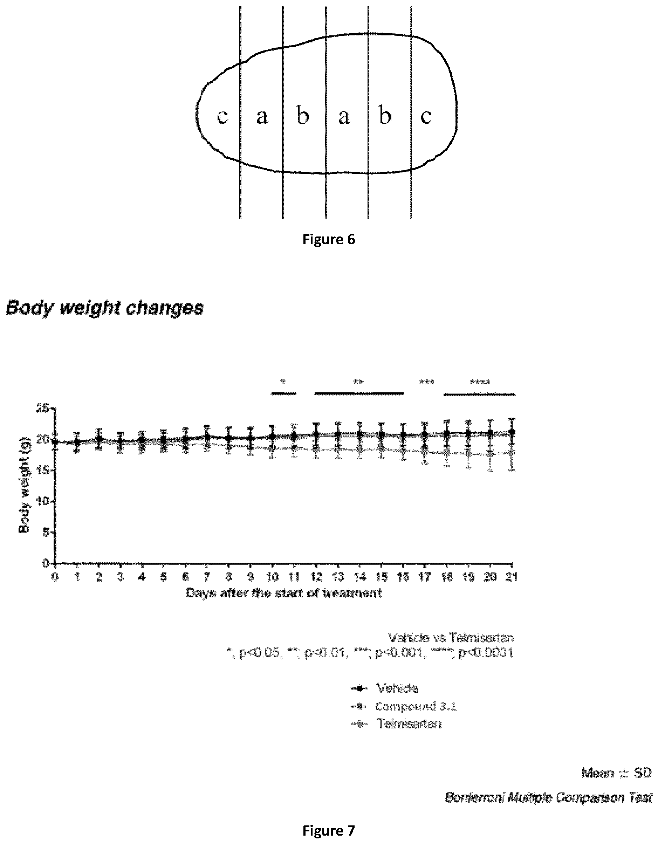

After 3 weeks of treatment mice were sacrificed, whole liver were collected and washed with cold saline. Individual whole liver (parietal side and visceral side) was taken photos. Liver weight were measured and liver-to-body weight ratio were calculated. The left lateral lobes of livers will be separated and dissected as described in Figure 6, and stored as described below:
a: Liver specimens were stored at -80°C embedded in Optimal Cutting Temperature (O.C.T., Sakura Finetek Japan, Japan) compound.
b: Liver specimens were fixed in Bouin's solution (Sigma-Aldrich, Japan) for 24 hours. After fixation, these specimens were proceeded to paraffin embedding for Sirius red-staining.
c: Liver specimens were snap frozen in liquid nitrogen and stored at -80°C.

Sections were cut from paraffin blocks of liver tissue using the rotary microtome (Leica Microsystems). After sectioning, each slide was coded a number for blind evaluation.

To visualize collagen deposition, Bouin's fixed liver sections were stained using picro-Sirius red solution (FUJIFILM Wako Pure Chemical Corporation). Briefly, sections were deparaffinized and hydrophilized with xylene, 100-70% alcohol series and RO water, and then treated with 0.03% picro-Sirius red solution (Cat No.: 194-16202) for 60 minutes. After washing through 0.5% acetic acid solution and RO water, stained sections were dehydrated and cleared with 70-100% alcohol series and xylene, then sealed with Entellan^{®} new (Merck, Germany) and used for observation.

For quantitative analysis of fibrosis area, bright field images of Sirius red-stained sections were captured around the central vein using a digital camera (DFC295; Leica, Germany) at 200-fold magnification, and the positive areas in 5 fields/section were measured using ImageJ. Statistical analyses were performed using Prism Software 6 (GraphPad Software, USA). Statistical analyses were performed using Bonferroni Multiple Comparison Test. Results were expressed as mean ± SD. Comparisons were made between the following groups; 1) Group 1 (Vehicle) vs. Group 2 (PhOx430); 2) Group 1 (Vehicle) vs. Group 3 (Telmisartan).

### Results

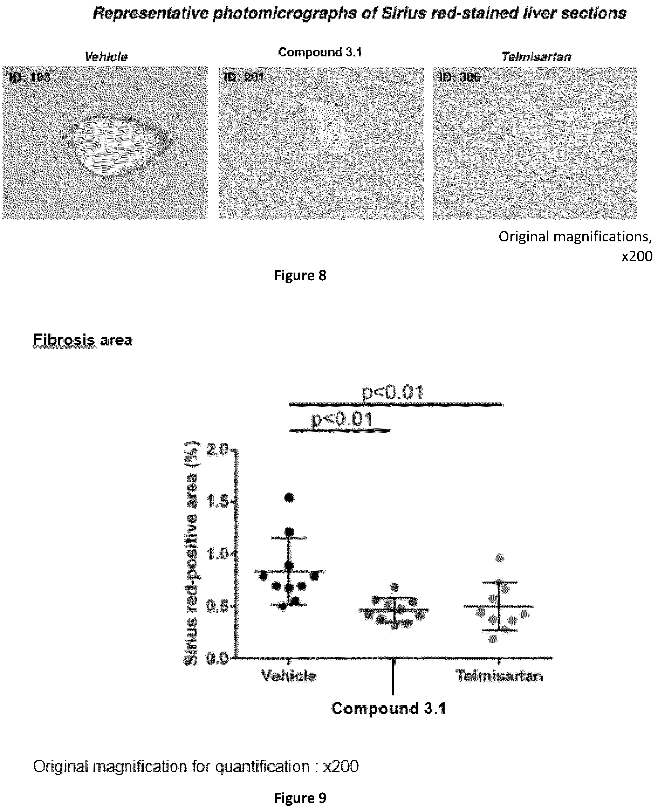

After 3 weeks of treatment no death, no body weight loss and no clinical signs have been observed in animals from Group2 treated by Compounds 3.1 (see Figure 7). Quantification of Sirius red stained liver sections demonstrates a clear and significant decrease of the fibrosis onset after oral administration of compound 3.1 nanosuspension in this mice model (see Figures 8 and 9).
Group1, Vehicle: Positive area 0,84 ± 0,31%
Group2, Compound 3.1: Positive area 0,47 ± 0,12%, p value < 0.01.
Group3, Telmisartan: Positive area 0,50 ± 0,24%, p value < 0.01.

## Claims

1. A compound of formula (1) for use in the treatment of fibrotic diseases, wherein the compound has the following formula (1): 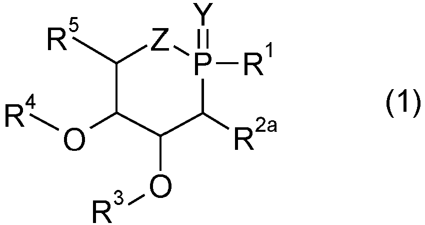
wherein Y represents an oxygen,
Z represents O, S, Se, NH or a NR₆ group, wherein R₆ is an aryl or an optionally substituted alkyl group, preferably an oxygen atom,
R₁ represents a hydrogen atom, an optionally substituted alkyl group or an aryl group,
R^{2a} represents a hydrogen atom, halogen, azide (N₃), a carbonate or dithiocarbonate group, a 1H-[1,2,3]triazolyl group or a group -X-R2, wherein
X represents an oxygen, a sulfur, a selenium atom, a NH or NR₇ group, R₇ being an optionally substituted aryl or alkyl group; X preferably represents O or NH, and;
R₂ represents an aryl group, an optionally substituted alkyl group, a hydrogen atom, a trichloroacetimidate group (-C(=NH)CCl₃), an acyl, formyl, sulfonyl, sulfinyl, tert-butyldiphenylsilyl, allyl group, a saccharyl, ester, amide, thioamide, sulfonamide group, or X-R₂ represents a P(O)R₂R₆ group, in which R₂ and R₆ represent independently from each other an aryl group, an optionally substituted alkyl group, OH, an alkoxy or an aryloxy group, R₃ and R₄ represent independently from each other an aryl, an optionally substituted alkyl group, an hydrogen atom, a trichloroacetimidate group, an acyl, formyl, sulfonyl, sulfinyl, tert-butyldiphenylsilyl group, an allyl, ester, amide, thioamide, sulfonamide group, or R₃ and R₄ taken together form a divalent radical of formula -R₃-R₄-, wherein -R₃-R₄- preferably represents an isopropylidene, benzylidene, diphenyl methylidene, cyclohexyl methylidene group, and their substituted analogues, for example a 4-methoxybenzylidene group, or a linear alkylene group such as an ethylene group,
R₅ represents a hydrogen atom or a hydrocarbon group comprising one or more heteroatoms, preferably selected from oxygen, sulfur or nitrogen, more preferably oxygen.

2. The compound for use according to claim 1, wherein the fibrotic disease is a lung, heart, liver, kidney, muscle, skin, soft tissue, bone marrow, intestinal, aortic or joint fibrosis.

3. The compound for use according to claim 1, wherein the fibrotic disease is a skin disease, kidney disease, pulmonary fibrosis, idiopathic pulmonary fibrosis, cystic fibrosis, endomyocardial fibrosis, mediastinal fibrosis, myelofibrosis, retroperitoneal fibrosis, progressive massive fibrosis, nephrogenic systemic fibrosis, Crohn's Disease, keloid, old myocardial infarction, scleroderma, systemic sclerosis, arthrofibrosis or an adhesive capsulitis.

4. The compound for use according to claim 1, wherein the fibrotic disease is a liver or kidney fibrosis.

5. The compound for use according to claim 1, wherein the fibrotic disease is an aortic fibrosis.

6. The compound for use according to claim 1, wherein the fibrotic disease is a skin fibrosis, including keloids or scleroderma.

7. The compound for use according to any one of claims 1-6, wherein the compound is of formula (1) with Y = Z = O.

8. The compound for use according to any one of claims 1-7, wherein R₅ is selected from the following groups: 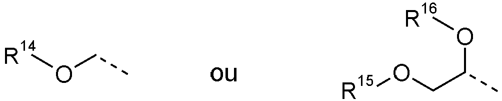 wherein R¹⁴, R¹⁵ and R¹⁶ represent, independantly from each other, a hydrogen atom, an aryl group, an optionally substituted alkyl group, a trichloroacetimidate group, an acyl, formyl, sulfonyl, sulfinyl, tert-butyldiphenylsilyl, allyl, ester, amide or a sulfonamide group, or R¹⁵ and R¹⁶, taken together, form a divalent radical of formula -R¹⁵-R¹⁶-, wherein -R¹⁵-R¹⁶- preferably represents an isopropylidene, benzylidene, diphenyl methylidene, or a cyclohexyl methylidene group, and their substituted analogues, for example a 4-methoxybenzylidene group, or a linear alkylene group, such as an ethylene group.

9. The compound for use according to any one of claims 1-8, wherein R1 is a phenyl group and/or X-R2 is OH and/or R3 and R4 represent a benzyl group.

10. The compound for use according to any one of claims 1-9, wherein the compound has the following formula (2) or (3): 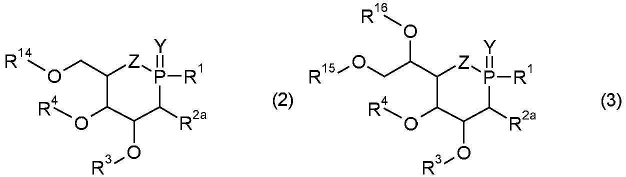 wherein R1, R2a, R3, R4, Y and Z are as defined in any one of claims 1, 8, 9 and 10, and R14, R15 and R16 represent, independently from each other, a hydrogen atom, an aryl, an optionally substituted alkyl group, a trichloroacetimidate group, an acyl, formyl, sulfonyl, sulfinyl, tert-butyldiphenylsilyl group, an allyl, ester, amide, thioamide, sulfonamide group, or R15 and R16, taken together, form a divalent radical of formula -R¹⁵-R¹⁶-, wherein -R¹⁵-R¹⁶- preferably represents an isopropylidene, benzylidene, diphenyl methylidene, cyclohexyl methylidene group, and their substituted analogues, for example a 4-methoxybenzylidene group, or a linear alkylene group such as an ethylene group, preferably R14 represents a benzyl group.

11. The compound for use according to any one of claims 1-10, wherein the compound of formula (1) is 3-Hydroxy-4,5-bis-benzyloxy-6-benzyloxymethyl-2-phenyl-2-oxo-2λ5-[1,2]oxaphosphinane.

12. The compound for use according to any one of the preceding claims, wherein the compound has the following Formula (I):

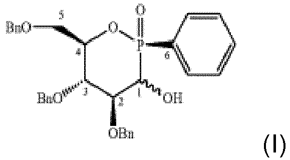

13. The compound for use according to any one of claims 1-12, wherein the treatment of fibrosis is by inhibition of the production of collagen fibers, more specifically collagen of types 1, 3 and/or 4, and/or by inhibition of mechanisms implicated in cell matrix and/or cell/cell interactions, including inhibition of fibroblast migration.

14. A kit comprising a compound of formula (1) as defined in anyone of claims 1 and 8-13, and instructions for use, for use in the treatment of fibrosis diseases.

## Patentansprüche

1. Eine Verbindung der Formel (1) zur Verwendung bei der Behandlung von fibrotischen Erkrankungen, wobei die Verbindung die folgende Formel (1) aufweist: 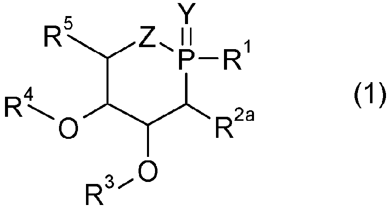
wobei Y ein Sauerstoff darstellt,
Z O, S, Se, NH oder eine NR₆-Gruppe darstellt, wobei R₆ eine Aryl- oder eine optional substituierte Alkylgruppe, vorzugsweise ein Sauerstoffatom, ist,
R₁ ein Wasserstoffatom, eine optional substituierte Alkylgruppe oder eine Arylgruppe darstellt,
R^{2a} ein Wasserstoffatom, ein Halogen, ein Azid (N₃), eine Carbonat- oder Dithiocarbonatgruppe, eine 1H-[1,2,3]Triazolylgruppe oder eine Gruppe -X-R2 darstellt, wobei
X ein Sauerstoff-, ein Schwefel- ein Selenatom, eine NH- oder NR₇-Gruppe darstellt, wobei R₇ eine optional substituierte Aryl- oder Alkylgruppe ist; X vorzugsweise O oder NH darstellt, und;
R₂ eine Arylgruppe, eine optional substituierte Alkylgruppe, ein Wasserstoffatom, eine Trichloracetimidatgruppe (-C(=NH)CCl₃), eine Acyl-, Formyl-, Sulfonyl-, Sulfinyl-, tert-Butyldiphenylsilyl- oder Allylgruppe, eine Saccharyl-, Ester-, Amid- oder Thioamid- oder Sulfonamidgruppe darstellt, oder X-R₂ eine P(O)R₂R₆-Gruppe darstellt, wobei R₂ und R₆ unabhängig voneinander eine Arylgruppe, eine optional substituierte Alkylgruppe, OH, eine Alkoxy- oder eine Aryloxygruppe darstten, R₃ und R₄ unabhängig voneinander eine Arylgruppe, eine optional substituierte Alkylgruppe, ein Wasserstoffatom, eine Trichloracetimidatgruppe, eine Acyl-, Formyl-, Sulfonyl-, Sulfinyl- oder tert-Butyldiphenylsilylgruppe, eine Allyl-, Ester, Amid-, Thioamid- oder Sulfonamidgruppe darstellen, oder R₃ und R₄ bilden zusammen ein zweiwertiges Radikal der Formel -R₃-R₄-, wobei -R₃-R₄- vorzugsweise eine Isopropyliden-, Benzyliden-, Diphenylmethyliden-, Cyclohexylmethyliden-Gruppe und deren substituierte Analoga, beispielsweise eine 4-Methoxybenzyliden-Gruppe, oder eine lineare Alkylengruppe wie eine Ethylengruppe darstellt,
R₅ ein Wasserstoffatom oder eine Kohlenwasserstoffgruppe darstellt, die ein oder mehrere Heteroatome umfasst, vorzugsweise ausgewählt aus Sauerstoff, Schwefel oder Stickstoff, noch bevorzugter Sauerstoff.

2. Die Verbindung zur Verwendung gemäß Anspruch 1, wobei es sich bei der fibrotischen Erkrankung um eine Fibrose der Lunge, des Herzens, der Leber, der Niere, der Muskulatur, der Haut, des Weichgewebes, des Knochenmarks, des Darms, der Aorta oder der Gelenke handelt.

3. Die Verbindung zur Verwendung gemäß Anspruch 1, wobei es sich bei der fibrotischen Erkrankung um eine Hauterkrankung, eine Nierenerkrankung, eine Lungenfibrose, eine idiopathische Lungenfibrose, eine zystische Fibrose, eine endomyokardiale Fibrose, eine mediastinale Fibrose, eine Myelofibrose, eine retroperitoneale Fibrose, eine progressive massive Fibrose, eine nephrogene systemische Fibrose, Morbus Crohn, ein Keloid, einen alten Myokardinfarkt, Sklerodermie, systemische Sklerose, Arthrofibrose oder eine adhäsive Kapselentzündung handelt.

4. Verbindung zur Verwendung gemäß Anspruch 1, wobei es sich bei der fibrotischen Erkrankung um eine Leber- oder Nierenfibrose handelt.

5. Verbindung zur Verwendung gemäß Anspruch 1, wobei es sich bei der fibrotischen Erkrankung um eine Aortenfibrose handelt.

6. Verbindung zur Verwendung gemäß Anspruch 1, wobei die fibrotische Erkrankung eine Hautfibrose ist, einschließlich Keloide oder Sklerodermie.

7. Verbindung zur Verwendung gemäß einem der Ansprüche 1 bis 6, wobei die Verbindung die Formel (1) aufweist, mit Y = Z = O.

8. Verbindung zur Verwendung gemäß einem der Ansprüche 1 bis 7, wobei R₅ aus den folgenden Gruppen ausgewählt ist: 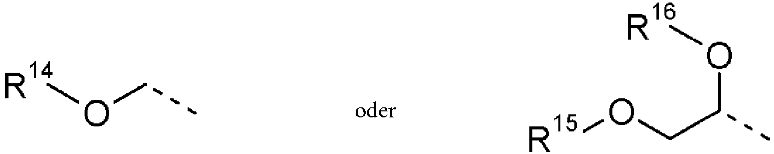 wobei R¹⁴, R¹⁵ und R¹⁶ unabhängig voneinander ein Wasserstoffatom, eine Arylgruppe, eine optional substituierte Alkylgruppe, eine Trichloracetimidatgruppe, eine Acyl-, Formyl-, Sulfonyl-, Sulfinyl-, tert-Butyldiphenylsilyl-, Allyl-, Ester-, Amid- oder eine Sulfonamidgruppe darstellen, oder R¹⁵ und R¹⁶ zusammen ein zweiwertiges Radikal der Formel -R¹⁵-R¹⁶-, wobei -R¹⁵-R¹⁶- vorzugsweise eine Isopropyliden-, Benzyliden-, Diphenylmethyliden- oder eine Cyclohexylmethylidengruppe sowie deren substituierte Analoga, beispielsweise eine 4-Methoxybenzylidengruppe, oder eine lineare Alkylengruppe, wie beispielsweise eine Ethylengruppe, darstellt.

9. Die Verbindung zur Verwendung gemäß einem der Ansprüche 1 bis 8, wobei R1 eine Phenylgruppe ist und/oder X-R2 OH ist und/oder R3 und R4 eine Benzylgruppe darstellen.

10. Die Verbindung zur Verwendung gemäß einem der Ansprüche 1 bis 9, wobei die Verbindung die folgende Formel (2) oder (3) aufweist: 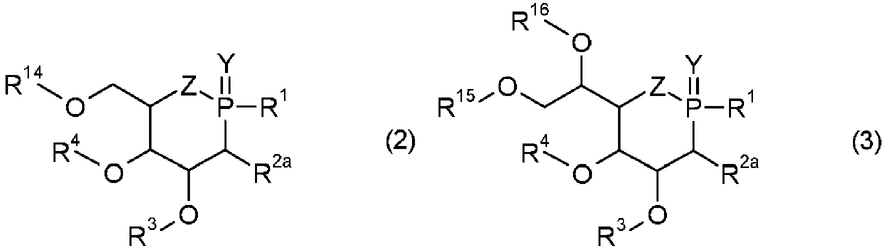 wobei R1, R2a, R3, R4, Y und Z wie in einem der Ansprüche 1, 8, 9 und 10 definiert sind und R14, R15 und R16 unabhängig voneinander ein Wasserstoffatom, ein Aryl, eine gegebenenfalls substituierte Alkylgruppe, eine Trichloracetimidatgruppe, eine Acyl-, Formyl-, Sulfonyl-, Sulfinyl- oder tert-Butyldiphenylsilylgruppe, eine Allyl-, Ester-, Amid-, Thioamid- oder Sulfonamidgruppe darstellen, oder R15 und R16 zusammen ein zweiwertiges Radikal der Formel -R¹⁵-R¹⁶- bilden, wobei -R¹⁵-R¹⁶- vorzugsweise eine Isopropyliden-, Benzyliden-, Diphenylmethyliden-, Cyclohexylmethyliden-Gruppe und deren substituierte Analoga, beispielsweise eine 4-Methoxybenzyliden-Gruppe, oder eine lineare Alkylengruppe wie eine Ethylengruppe darstellt, wobei R14 vorzugsweise eine Benzylgruppe darstellt.

11. Die Verbindung zur Verwendung gemäß einem der Ansprüche 1-10, wobei die Verbindung der Formel (1) 3-Hydroxy-4,5-bis-benzyloxy-6-benzyloxymethyl-2-phenyl-2-oxo-2λ5-[1,2]oxaphosphinan ist.

12. Die Verbindung zur Verwendung gemäß einem der vorstehenden Ansprüche, wobei die Verbindung die folgende Formel (I) aufweist:

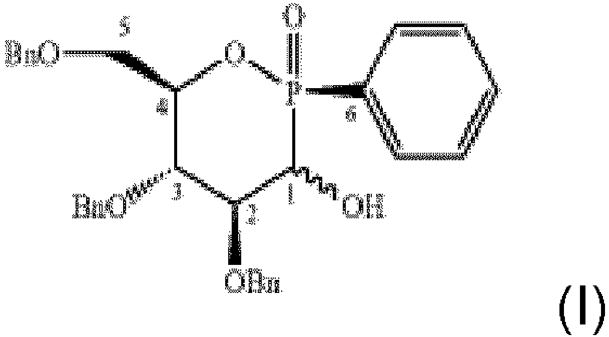

13. Die Verbindung zur Verwendung gemäß einem der Ansprüche 1 bis 12, wobei die Behandlung von Fibrose durch Hemmung der Bildung von Kollagenfasern, insbesondere von Kollagen der Typen 1, 3 und/oder 4, und/oder durch Hemmung von Mechanismen erfolgt, die an Zell-Matrix- und/oder Zell/Zell-Wechselwirkungen beteiligt sind, einschließlich der Hemmung der Fibroblastenmigration.

14. Ein Kit umfassend eine Verbindung der Formel (1), wie in einem der Ansprüche 1 und 8-13 definiert, sowie eine Gebrauchsanweisung, zur Verwendung in der Behandlung von Fibroseerkrankungen.

## Revendications

1. Composé de formule (1) pour son utilisation dans le traitement des maladies fibrotiques, où le composé a la formule (1) suivante : 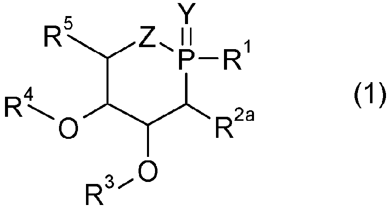
dans laquelle Y représente un oxygène,
Z représente O, S, Se, NH ou un groupe NR₆, où R₆ est un groupe aryle ou un groupe alkyle éventuellement substitué, de préférence un atome d'oxygène,
R₁ représente un atome d'hydrogène, un groupe alkyle éventuellement substitué ou un groupe aryle,
R^{2a} représente un atome d'hydrogène, un halogène, un azide (N₃), un groupe carbonate ou dithiocarbonate, un groupe 1H-[1,2,3]triazolyle ou un groupe -X-R2, où
X représente un atome d'oxygène, un atome de soufre, un atome de sélénium, un groupe NH ou NR₇, R₇ étant un groupe aryle ou alkyle éventuellement substitué ; X représente de préférence O ou NH, et ;
R₂ représente un groupe aryle, un groupe alkyle éventuellement substitué, un atome d'hydrogène, un groupe trichloroacétimidate (-C(=NH)CCl₃), un groupe acyle, formyle, sulfonyle, sulfinyle, tert.-butyldiphénylsilyle, allyle, un groupe saccharyle, ester, amide, thioamide, sulfonamide, ou X-R₂ représente un groupe P(O)R₂R₆, dans lequel R₂ et R₆ représentent indépendamment les uns des autres un groupe aryle, un groupe alkyle éventuellement substitué, OH, un groupe alcoxy ou aryloxy, R₃ et R₄ représentent indépendamment les uns des autres un groupe aryle, un groupe alkyle éventuellement substitué, un atome d'hydrogène, un groupe trichloroacétimidate, un groupe acyle, formyle, sulfonyle, sulfinyle, tert.-butyldiphénylsilyle, un groupe allyle, ester, amide, thioamide, sulfonamide, ou R₃ et R₄ pris conjointement forment un radical divalent de formule -R₃-R₄-, où -R₃-R₄- représente de préférence un groupe isopropylidène, benzylidène, diphényl méthylidène, cyclohexyl méthylidène, et leurs analogues substitués, par exemple un groupe 4-méthoxybenzylidène, ou un groupe alkylène linéaire comme un groupe éthylène,
R₅ représente un atome d'hydrogène ou un groupe hydrocarboné comprenant un ou plusieurs hétéroatomes, de préférence choisis parmi oxygène, soufre ou azote, plus préférentiellement oxygène.

2. Composé pour son utilisation selon la revendication 1, dans lequel la maladie fibrotique est une fibrose pulmonaire, cardiaque, hépatique, rénale, musculaire, cutanée, des tissus mous, de la moelle osseuse, intestinale, aortique ou articulaire.

3. Composé pour son utilisation selon la revendication 1, dans lequel la maladie fibrotique est une maladie cutanée, une maladie rénale, une fibrose pulmonaire, une fibrose pulmonaire idiopathique, une fibrose kystique, une fibrose endomyocardique, une fibrose médiastinale, une myélofibrose, une fibrose rétropéritonéale, une fibrose massive progressive, une fibrose systémique néphrogénique, la maladie de Crohn, une kéloïde, un ancien infarctus myocardique, une sclérodermie, une sclérose systémique, l'arthrofibrose ou une capsulite adhésive.

4. Composé pour son utilisation selon la revendication 1, dans lequel la maladie fibrotique est une fibrose hépatique ou rénale.

5. Composé pour son utilisation selon la revendication 1, dans lequel la maladie fibrotique est une fibrose aortique.

6. Composé pour son utilisation selon la revendication 1, dans lequel la maladie fibrotique est une fibrose cutanée, y compris des kéloïdes ou la sclérodermie.

7. Composé pour son utilisation selon l'une quelconque des revendications 1 à 6, où le composé est de formule (1) avec Y = Z = O.

8. Composé pour son utilisation selon l'une quelconque des revendications 1 à 7, où R₅ est choisi dans les groupes suivants : 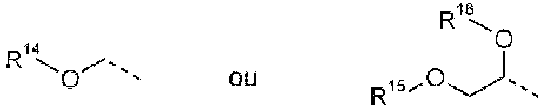 où R¹⁴, R¹⁵ et R¹⁶ représentent indépendamment les uns des autres un atome d'hydrogène, un groupe aryle, un groupe alkyle éventuellement substitué, un groupe trichloroacétimidate, un groupe acyle, formyle, sulfonyle, sulfinyle, tert.-butyldiphénylsilyle, allyle, ester, amide, ou sulfonamide, ou R¹⁵ et R¹⁶, pris conjointement, forment un radical divalent de formule -R¹⁵-R¹⁶-, où -R¹⁵-R¹⁶-représente de préférence un groupe isopropylidène, benzylidène, diphényl méthylidène, ou cyclohexyl méthylidène, et leurs analogues substitués, par exemple un groupe 4-méthoxybenzylidène, ou un groupe alkylène linéaire comme un groupe éthylène.

9. Composé pour son utilisation selon l'une quelconque des revendications 1 à 8, dans lequel R1 est un groupe phényle et/ou X-R2 est OH et/ou R3 et R4 représentent un groupe benzyle.

10. Composé pour son utilisation selon l'une quelconque des revendications 1 à 9, où le composé a la formule suivante (2) ou (3) : 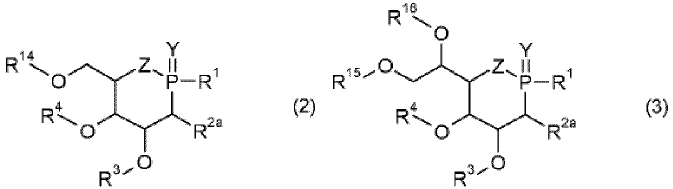 dans lesquelles R1, R2a, R3, R4, Y et Z sont tels que définis selon l'une quelconque des revendications 1, 8, 9 et 10, et R14, R15 et R16 représentent indépendamment les uns des autres un atome d'hydrogène, un groupe aryle, un groupe alkyle éventuellement substitué, un groupe trichloroacétimidate, un groupe acyle, formyle, sulfonyle, sulfinyle, tert.-butyldiphénylsilyle, un groupe allyle, ester, amide, thioamide, sulfonamide, ou R15 et R16, pris conjointement, forment un radical divalent de formule -R¹⁵-R¹⁶-, où -R¹⁵-R¹⁶- représente de préférence un groupe isopropylidène, benzylidène, diphényl méthylidène, cyclohexyl méthylidène, et leurs analogues substitués, par exemple un groupe 4-méthoxybenzylidène, ou un groupe alkylène linéaire comme un groupe éthylène, de préférence R14 représente un groupe benzyle.

11. Composé pour son utilisation selon l'une quelconque des revendications 1 à 10, où le composé de formule (1) est le 3-hydroxy-4,5-bis-benzyloxy-6-benzyloxyméthyl-2-phényl-2-oxo-2λ5-[1,2]oxaphosphinane.

12. Composé pour son utilisation selon l'une quelconque des revendications précédentes, où le composé présente la formule (I) suivante :

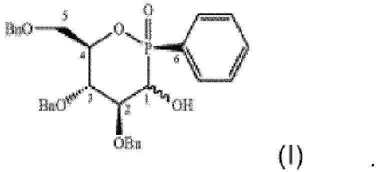

13. Composé pour son utilisation selon l'une quelconque des revendications 1 à 12, dans lequel le traitement de la fibrose se fait par inhibition de la production des fibres de collagène, plus spécifiquement du collagène des types 1, 3 et/ou 4, et/ou par inhibition des mécanismes impliqués dans la matrice cellulaire et/ou des interactions cellule/cellule, y compris l'inhibition de la migration des fibroblastes.

14. Kit comprenant un composé de formule (1) tel que défini selon l'une quelconque des revendications 1 et 8 à 13, et des instructions d'utilisation, pour son utilisation dans le traitement des maladies fibrotiques.
